(19) Europäisches Patentamt
European Patent Office
Office européen des brevets

(11) **EP 4 253 952 B1**

(12) **EUROPÄISCHE PATENTSCHRIFT**

(45) Veröffentlichungstag und Bekanntmachung des Hinweises auf die Patenterteilung:
**02.07.2025   Patentblatt 2025/27**

(21) Anmeldenummer: **23000016.8**

(22) Anmeldetag: **01.02.2023**

(51) Internationale Patentklassifikation (IPC):
**G01N 33/00** (2006.01)   **G01N 33/24** (2006.01)

(52) Gemeinsame Patentklassifikation (CPC):
**G01N 33/24; G01N 33/245;** G01N 33/0037;
G01N 33/0054

(54) **VERFAHREN ZUR QUALITATIVEN UND QUANTITATIVEN IN-SITU ECHTZEITERMITTLUNG DES AMMONIUM-GEHALTS UND DES AMMONIUM-UMSATZES ZU NITRIT IM BODEN**

METHOD FOR THE QUALITATIVE AND QUANTITATIVE IN-SITU REAL-TIME DETERMINATION OF THE AMMONIUM CONTENT AND THE AMMONIUM CONVERSION TO NITRITE IN SOIL

PROCÉDÉ DE DÉTERMINATION QUALITATIVE ET QUANTITATIVE EN TEMPS RÉEL DE LA TENEUR EN AMMONIUM ET DE LA CONVERSION EN NITRITE D'AMMONIUM DANS LE SOL

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **30.03.2022   DE 102022001089**

(43) Veröffentlichungstag der Anmeldung:
**04.10.2023   Patentblatt 2023/40**

(73) Patentinhaber: **Forschungszentrum Jülich GmbH**
**52425 Jülich (DE)**

(72) Erfinder:
• **Brüggemann, Nicolas**
  **52393 Hürtgenwald (DE)**
• **Claß, Matthias**
  **52351 Düren (DE)**
• **Herbst, Michael**
  **52428 Jülich (DE)**

(56) Entgegenhaltungen:
• **WALKER JOHN T ET AL: "Nitrogen trace gas emissions from a riparian ecosystem in southern Appalachia", CHEMOSPHERE, vol. 49, no. 10, 2 November 2002 (2002-11-02), GB, pages 1389 - 1398, XP093068114, ISSN: 0045-6535, DOI: 10.1016/S0045-6535(02)00320-X**
• **PACIFICO FEDERICA ET AL: "Measurements of nitric oxide and ammonia soil fluxes from a wet savanna ecosystem site in West Africa during the DACCIWA field campaign", ATMOSPHERIC CHEMISTRY AND PHYSICS, vol. 19, no. 4, 21 February 2019 (2019-02-21), pages 2299 - 2325, XP093068200, DOI: 10.5194/acp-19-2299-2019**

**Beschreibung**

Die Erfindung betrifft ein Verfahren zur qualitativen und quantitativen In-situ Echtzeitermittlung des Ammonium-Gehalts und des Ammonium-Umsatzes zu Nitrit im Boden.

Auf Grund gesetzlicher Vorgaben ist es in der Landwirtschaft erforderlich, den Zustand des Bodens, insbesondere hinsichtlich des mineralischen Stickstoffgehalts, zu überwachen und zu kontrollieren, um zum einen eine Überdüngung des Bodens zu vermeiden und zum anderen einen Eintrag von Nitrat ins Grundwasser zu verhindern. Nitrat wird im Boden durch den mikrobiellen Prozess der Nitrifikation aus Ammonium über die Zwischenstufe Nitrit gebildet. Für die Überwachung der Böden und Gewässer werden nach den derzeit bekannten Verfahren punktuell Bodenproben entnommen, woraus anschließend im Labor Ammonium und Nitrat extrahiert und deren Konzentration bestimmt werden. Die Bodenproben repräsentieren dabei nur punktuelle Werte, die später gemittelt werden. Von der Probenahme bis zum Ergebnis ist viel Arbeitszeit in Form von Probennahme, Transport und Laboranalytik aufzuwenden.

Aus dem Stand der Technik (US 16/466,128) ist beispielsweise eine Messmethode bekannt, bei der eine Messung von Ammonium im Porenwasser des Bodens mit Hilfe von Sonden erfolgt, die an unterschiedlichen Positionen direkt in den Boden eingebracht werden und in der Lage sind, die gewünschten Analyten direkt auszuwerten. Eine Aussage über den Ammonium-Gehalt des Bodens ist daher nur für das Porenwasser in unmittelbarer Nähe der Sonde gültig. Es könnten zwar eine Vielzahl an Sonden in kurzen Abständen in den Boden eingebracht werden, jedoch würde dann der finanzielle und technische Aufwand dementsprechend ansteigen. Zudem wäre eine mobile Anwendung nicht möglich. Des Weiteren erfordert der Einsatz von stationären Sonden ausreichend Porenwasser, damit diese ausreichend mit Wasser umhüllt / benetzt sind.

Walker John T ET AL: "Nitrogen trace gas emissions from a riparian ecosystem in southern Appalachia", Chemosphere, Bd. 49, Nr. 10, 2. November 2002, Seiten 1389-1398, XP093068114, ISSN: 0045-6535, DOI: 10.1016/S0045-6535(02)00320-X offenbart einen Zusammenhang zwischen der Ausgasungsrate von Stickstoffmonoxid und des Ammoniumgehalts des Erdbodens.

Die bisher bekannten Verfahren sind daher nachteiligerweise zum einen zeitintensiv und zum anderen stellen sie eine destruktive Methode dar. Die Methoden gemäß Stand der Technik ermöglichen auf Grund ihrer punktuellen Probenahme und der gemittelten Bestimmung keine Echtzeitmessung vor Ort und keine räumlich hochaufgelöste Bestimmung des mineralischen Stickstoffgehalts des Bodens, so dass eine spezifisch an den lokalen Bedarf angepasste Düngung oder eine entsprechende Applikation beispielsweise von Nitrifikationsinhibitoren nicht möglich ist.

Es ist daher die Aufgabe der Erfindung, die Nachteile des Stands der Technik zu überwinden und ein Verfahren zur Verfügung zu stellen, mit dem direkt vor Ort (In-situ) eine räumlich hochaufgelöste qualitative und quantitative Echtzeitbestimmung des Ammonium-Gehalts des Bodens und des Ammonium-Umsatzes zu Nitrit im Boden ermöglicht wird. Unter der Bezeichnung "Boden" wird im Rahmen der vorliegenden Erfindung der obere Bodenbereich (0-30 cm) verstanden, der in der Landwirtschaft typischerweise den durch Bodenbearbeitung und Düngung maßgeblich beeinflussten Bereich darstellt.

Die Aufgaben der Erfindung werden gelöst durch ein Verfahren mit den Kennzeichen gemäß Hauptanspruch. Vorteilhafte Ausführungsformen des Verfahrens sind den jeweils darauf rückbezogenen Ansprüchen zu entnehmen.

Die Erfinder haben in überraschender Weise festgestellt, dass die Bestimmung des Stickstoffstatus, im Folgenden vereinfacht mit "N-Status" bezeichnet, des Bodens über Bodenausgasungen möglich ist. In vorteilhafter Weise werden mit der vorliegenden Erfindung für die jeweilige lokale Messung des N-Status des Bodens keine Sonden in den Boden eingebracht, so dass die Messung somit nicht-invasiv erfolgt und weiterhin damit eine mobile Anwendung zur sensorischen Erfassung großer Flächen ermöglicht wird.

Die hier offenbarte Messanordnung zur qualitativen und quantitativen In-Situ- und Echtzeitermittlung des Ammonium-Gehalts und des Ammonium-Umsatzes zu Nitrit im Boden durch räumlich hochaufgelöste, qualitative und quantitative Bestimmung umfasst dabei folgende Komponenten:

- eine oder mehrere Gasentnahmevorrichtung/en
- wenigstens ein Multigasmessgerät
- wenigstens ein Massenflussgerät
- wenigstens eine Auslesevorrichtung sowie
- wenigstens eine Auswertevorrichtung für das Multigasmessgerät

Die Gasentnahmevorrichtung kann eine Pumpe umfassen, welche, abhängig von der Skalierung des Messgeräts, die Pumpe eines Multigasmessgerätes sein kann, oder, bei größeren Anwendungen, eine separate Pumpe, die dann an ein Multigasmessgerät angeschlossen ist. Die Gasentnahmevorrichtung umfasst dabei weiterhin eine Messhaube, die zur Entnahme und Aufnahme der Bodenausgasung entweder oberhalb des Bodens gehalten oder mit ihrem unteren Rand gasdicht auf den Boden aufgesetzt wird. Bei einem gasdichten Aufsetzen der Messhaube auf den Boden, kann vorteilhaft eine gasdichte Trennung der Bodenausgasungen von der Umgebungsluft erfolgen. Die Gasentnahmevorrichtung kann auch mittels eines automatischen Transportsystems auf den Boden aufgesetzt werden. Die Form der Messhaube kann beispielsweise glockenartig ausgestaltet sein, wobei auch andere Ausgestaltungen der Form der Messhaube möglich sind, wie beispielsweise Hohlkörper mit einem runden, ovalen oder polygonalen Querschnitt. Messhaube, Pumpe und Multigasmessgerät sind durch eine Gasleitung miteinander verbunden, über welche die zu messenden Bodenausgasungen aus der Messhaube zum Multigasmessgerät geführt werden.

Die Fläche des Bodens, die mit der Gasentnahmevorrichtung erfasst wird, ist abhängig von dem Durchmesser oder den Abmessungen der Messhaube und damit der Fläche, mit der die Messhaube den Boden abdeckt. Diese Fläche beträgt typischerweise einige hundert Quadratzentimeter.

Die von der Gasentnahmevorrichtung erfasste Bodentiefe umfasst mindestens den Bodenhorizont 0-30 cm. Das ist die Bodentiefe, durch welche die landwirtschaftliche Bearbeitung den Boden am stärksten beeinflusst und die für das Pflanzenwachstum und die Stickstoffumsetzungen der relevanteste Bodenbereich ist.

Zur Gasentnahme erzeugt die Pumpe einen konstanten Durchfluss von Luft durch die Messhaube. Der kontrollierte Luftfluss durch die Messhaube wird erreicht, indem Umgebungsluft durch eine sich oberhalb der Messhaube befindende Einlassöffnung angesaugt und durch die Messhaube geleitet wird. Dort wird die Umgebungsluft mit den vom Boden abgegeben Gasen beladen und dann über eine separate Auslassöffnung über eine Schlauchleitung zum Multigasmessgerät geleitet, wo die Luft aus der Messhaube auf die Konzentration der in ihr enthaltenen Spurengase analysiert wird (s.u.). Hierbei ist durch entsprechendes Design der Messhaube darauf zu achten, dass dabei innerhalb der Messhaube kein Über- oder Unterdruck entsteht. In regelmäßigen Abständen wird auch die Gaskonzentration der Umgebungsluft selbst bestimmt. Aus den Gaskonzentrationen der Umgebungsluft vor Eintritt in die bzw. nach Austritt aus der Messhaube und der Durchflussrate von Luft durch die Messhaube wird dann die Menge der vom Boden an der Oberfläche abgegebenen Gase bestimmt.

Das Multigasmessgerät sollte wenigstens zwei Analysekanäle aufweisen, um die Mindestanzahl der Gase, die für die Bestimmung des Ammonium-Gehalts und des Ammonium-Umsatzes zu Nitrit erforderlich sind, auswerten zu können. In der vorliegenden Messanordnung und dem Verfahren werden zu diesem Zweck mit dem Multigasmessgerät die Gase $H_2O$ (Wasserdampf), $NH_3$ und NO in einer simultanen Echtzeitmessung erfasst.

Bei dem Multigasmessgerät handelt sich um einen Infrarotabsorptionsanalysator, welcher auf die gewünschten spezifischen Absorptionsbanden der zu messenden Gaskomponenten im mittleren Infrarotspektralbereich eingestellt ist. Das Multigasmessgerät selbst misst nur Gase, also keine Ionen, jedoch stehen die Ionen im Boden in Wechselwirkung mit den emittierten Gasen. Zur Einstellung des Gerätenullpunktes des Multigasmessgeräts erfolgt in regelmäßigen Abständen eine Messung von trockener synthetischer Luft oder trockenem Stickstoff.

Die mit dem Multigasmessgerät gemessenen Gaskomponenten $NH_3$ und NO stehen in Wechselwirkung mit folgenden Stickstoffverbindungen im Boden:

$$1.) \qquad NH_3 \text{ (gasförmig)} \Leftrightarrow NH_4^+ \text{ (Ammonium im Boden)}$$

$$2.) \qquad NO \text{ (gasförmig)} \Leftrightarrow NO_2^- \text{ (Nitrit im Boden).}$$

Dabei wird die Wechselwirkung zwischen Gasen und Ionen im Boden durch den pH-Wert, die Temperatur, den Wassergehalt und die Dichte des Bodens sowie durch dessen Kationen- und Anionen-Austauschkapazität beeinflusst.

Die Messanordnung kann in einer vorteilhaften Ausgestaltung wenigstens noch eine der Komponenten pH-Sensor, Temperatursensor und/oder Bodenwassergehaltsensor umfassen. Die Messdaten weiterer Sensoren, insbesondere pH-Sensoren, Temperatursensoren, Bodenwassergehaltssensoren, die in den Boden während eines Messvorgangs temporär eingeführt werden, können vorteilhaft mit zuvor für den Boden ermittelten Kalibrationsdaten verglichen werden und als Parameter bei der rechnerischen Bestimmung des Ammonium- und des Nitrit-Gehalts gemäß Verfahrensschritt d) berücksichtigt werden.

Bodenfeuchte, Temperatur und pH-Wert des Bodens sind wichtige Parameter, die die Korrelation des Ammonium- und Nitrit-Gehalts im Boden und der Ammoniak- und Stickoxid-Ausgasung des Bodens in Relation beeinflussen. Ein Bodenwassergehaltssensor, ein Temperatursensor und ein pH-Sensor werden daher während eines Messvorgangs temporär in den Boden eingeführt, so dass diese Parameter parallel mit der Entnahme der Bodenausgasung bestimmt werden können. Für die qualitative und quantitative Bestimmung der Bodenausgasungen spielt auch die Porosität des Bodens bei der Ausgasung eine wichtige Rolle. Daher ist es vorteilhaft, dass in entsprechenden Parametrisierungsversuchen Transferfunktionen ermittelt werden, mit denen die für die jeweiligen Bodenbedingungen (Temperatur, Wassergehalt, Dichte) die entsprechenden Transferkoeffizienten bestimmt werden können.

Die Zusammensetzung der zum Multigasmessgerät geleiteten Bodenausgasungen wird direkt vor Ort vom Multigasmessgerät quantitativ analysiert. Anschließend werden die ermittelten Gaskonzentrationen zusammen mit der vom angeschlossenem Massenflussmessgerät ermittelten Luftdurchflussrate durch die Gasentnahmevorrichtung, insbesondere die Messhaube, mithilfe von Kalibrationsmessungen aus einer Referenzdatenbank prozessiert und in die Konzentrationen der entsprechenden Ionen ($NH_3 \rightarrow NH_a^+$ bzw. $NO \rightarrow NO_2^-$) im Boden umgerechnet. Der Umsatz von Ammonium zu Nitrit kann anhand der Stickstoffmonoxid-Konzentration der Bodenausgasungen ermittelt werden, die im proportionalen Verhältnis zur Nitritkonzentration im Boden steht. Die Nitritkonzentration im Boden wiederum steht in direktem Verhältnis zum Umsatz von Ammonium zum Nitrit.

Die erfassten Daten können in einem internen Speicher des Multigasmessgeräts abgespeichert werden. Die Referenzdaten zur Umrechnung der gemessenen Gaskonzentrationen in die Konzentration der mit den Gasen in Wechselwirkung stehenden Ionen im Boden werden durch vorherige Messungen, die der Kalibration dienen, generiert.

Als Massenflussmessgeräte können alle nach dem Stand der Technik bekannten Geräte eingesetzt werden.

Der Messbereich des Massenflussmessgeräts sollte idealerweise im Bereich der geeigneten Durchflussrate durch die Messhaube der Gasentnahmevorrichtung liegen, die wiederum von den Dimensionen der Messhaube abhängt. Wenn beispielsweise mit einer Rate von 1 l/min Bodenausgasungen aus der Messhaube entnommen wird, muss das Massenflussmessgeräts Luftdurchflussraten im Bereich von 1 l/min zuverlässig bestimmen können.

Die Auslesevorrichtung sollte vorzugsweise in der Lage sein, alle nötige Daten (Gaskonzentrationen, Standortort des Messgeräts, Umweltfaktoren etc....) zu erfassen, zu bearbeiten und zu verrechnen. Hierzu wäre beispielsweise ein kleiner Computer/Rechner mit der nötigen Software (Modellierungsprogramm und weitere Programme zum Auslesen der Sensoren etc.) geeignet.

Die Auslesevorrichtung bündelt alle Datensätze (Bodengaskonzentrationen, Standort des Messgeräts, Lufttemperatur, Bodentemperatur etc.) und bereitet sie für die Auswertevorrichtung auf.

Die Auswertevorrichtung errechnet mit den vorhandenen Daten den Ammonium-Pool sowie die Ammonium-Umsetzungsrate des Bodens.

Eine flächendeckende Messung des Bodens kann dadurch erfolgen, dass beispielsweise - abhängig von der gewünschten Auflösung - an zuvor bestimmten Gasentnahmestellen für die Bodenausgasungen Messungen durchgeführt werden, wobei die Gasentnahmevorrichtungen jeweils an den zuvor bestimmten Gasentnahmestellen angeordnet sind und die Bodenausgasungsproben wenigstens einem Multigasmessgerät, zum Beispiel über eine Pumpe, zugeführt werden.

Alternativ können auch kontinuierliche Messungen über die ganze zu messende Bodenfläche durchgeführt werden, wobei die Messanordnung dazu mobil ausgestaltet ist, indem die gesamte Messanordnung und/oder wenigstens eine und/oder mehrere Komponenten der Messanordnung auf einem Fahrzeug, das auch ein Traktor oder ein anderes in der Landwirtschaft üblicherweise eingesetztes mobiles Gerät sein kann, angeordnet sind. So kann dann mit Hilfe beispielsweise einer autonom fahrbaren mobilen Anordnung, z.B. mit Hilfe eines Fahrzeugs, die Messanordnung über die zu messende Fläche des Bodens bewegt werden und dabei jeweils an definierten lokalen Positionen des Bodens eine Probe der Bodenausgasungen mit Hilfe der Gasentnahmevorrichtung entnommen und analysiert werden.

Die Messanordnung kann in einer vorteilhaften Ausführung auch eine mobile Stromversorgung, insbesondere mobile Batterie-Einheiten und/oder eine an die Batterie des Fahrzeugs der Messanordnung angeschlossene Stromversorgung und Zwischenschaltung eines DC/AC-Wandlers umfassen.

Die Bestimmung der räumlichen Zuordnung der Ergebnisse der Bodenausgasungen kann beispielsweise über GPS-basierte Positionsbestimmung- und Navigationssysteme erfolgen, die in einer weiteren vorteilhaften Ausführung der Messanordnung ebenfalls weitere Komponenten dieser Messanordnung sein können.

Im Folgenden soll das erfindungsgemäße Verfahren in seiner allgemeinen Form beschrieben werden.

Das Verfahren zur qualitativen und quantitativen In-situ- und Echtzeitermittlung des Ammonium-Gehalts und des Ammonium-Umsatzes zu Nitrit im Boden, bei der die zuvor beschriebene Messanordnung eingesetzt wird, umfasst wenigstens folgende Verfahrensschritte:

a) Entnahme von Bodenausgasungen eines Bodens mittels einer oder mehrerer Gasentnahmevorrichtung/en, durch beispielsweise Ansaugen der Bodenausgasungen, und Beförderung dieser Bodenausgasungen zu wenigstens einem Multigasmessgerät,

b) Bestimmung des Massenflusses der Bodenausgasungen zum Multigasmessgerät anhand eines Massenflussmessgeräts,

c) Bestimmung der Ausgasungsraten $f$ von $NH_3$ und NO aus dem Boden mittels eines Multigasmessgeräts und einer Auslesevorrichtung, bezogen auf die von der Gasentnahmevorrichtung abgedeckte Fläche des Bodens,

d) Rechnerische Bestimmung des Ammonium- und des Nitrit-Gehalts des Bodens durch eine Auswertevorrichtung, wobei für die Bestimmung des Ammonium-Gehalts die ermittelte Ausgasungsrate f von $NH_3$ und für die Bestimmung des Nitrit-Gehalts die ermittelte Ausgasungsrate f von NO aus Schritt c) mit zuvor für den Boden jeweils ermittelten Kalibrationsdaten verglichen wird und so In-situ die lokale Konzentration des Ammonium- und des Nitrit-Gehalts des Bodens quantitativ sowie der Umsatz von Ammonium zu Nitrit qualitativ in Echtzeit bestimmt werden.

Einzelne ausgewählte Komponenten der Messanordnung, insbesondere die Gasentnahmevorrichtungen und das Multigasmessgerät, können beispielsweise entlang eines vorgegebenen Rasters oder Wegpunktemusters über das zu messende Feld bzw. die Bodenfläche bewegt werden, um Messdaten zu den Konzentrationen der Bodengase an einer Vielzahl von Punkten auf der Fläche des Bodens zu entnehmen oder sie werden an einer Vielzahl von lokal bestimmten Punkten auf der Fläche des Bodens zu einer kontinuierlichen Messung eingesetzt. Die Entnahme von Bodenausgasungen erfolgt dabei an wenigstens zwei unterschiedlichen Positionen des Bodens mit Hilfe von wenigstens zwei Gasentnahmevorrichtungen. Je nach gewünschter lokaler Auflösung der zu erfassenden Daten hinsichtlich des Ammonium-Gehalts und des Ammonium-Umsatzes zu Nitrit im Boden kann auch eine Entnahme der Bodenausgasungen mit einer entsprechenden Anzahl an Gasentnahmevorrichtungen erfolgen.

In einer vorteilhaften Ausführung des Verfahrens, kann die mobil ausgestaltete Messanordnung und/oder eine oder mehrere oder alle Komponenten der Messanordnung, an unterschiedliche Positionen des Bodens bewegt werden.

Da Bodenfeuchte, Temperatur und pH-Wert des Bodens wichtige Parameter sind, die die Korrelation des Ammonium- und Nitrit-Gehalts im Boden und der Ammoniak- und Stickoxid-Ausgasung des Bodens in Relation beeinflussen, werden in einer vorteilhaften Ausführung des Verfahrens Messdaten weiterer Sensoren, insbesondere pH-Sensoren, Temperatursensoren, Bodenwassergehaltssensoren, die in den Boden während eines Messvorgangs temporär eingeführt werden, mit zuvor für den Boden ermittelten Kalibrationsdaten verglichen und als Parameter bei der rechnerischen Bestimmung des Ammonium- und des Nitrit-Gehalts gemäß Schritt d) berücksichtigt.

Die Messdaten werden mit Hilfe des Multigasmessgeräts qualitativ ausgelesen und unmittelbar in Konzentrationen der Bodenkomponenten Ammonium und Nitrit umgerechnet. Daraus lassen sich beispielsweise Empfehlungen zur Düngung bzw. zur Applikation von Nitrifikationsinhibitoren generieren. Eine Datenübertragung vor Ort zur Auslesevorrichtung, Auswertevorrichtung und einer daran möglicherweise angeschlossenen weiteren Steuerungsvorrichtung, kann kabelgebunden (USB) oder auch drahtlos (WLAN) erfolgen. Die Ergebnisse aus Verfahrensschritt d) können auch extern, zum Beispiel in einer Daten-Cloud, gespeichert und von dort aus wieder abgerufen werden.

Die Figuren zeigen die Messanordnung und durch das erfindungsgemäße Verfahren gewonnene experimentelle Ergebnisse sowie Grundlagen der Erfindung.

Figur 1: Schematische Darstellung eines Beispiels der Messanordnung als Blockschaltbild

Figur 2: Fahrzeug mit Messanordnung

Figur 3: Ergebnisse der gemessenen $NH_3$-Ausgasungsraten $f_{NH3,t}$ aus dem Boden und des daraus mittels einer Modellierungssoftware (AgroC) errechneten Ammoniumgehalts des Bodens

Figur 4: Analytisch aus Bodenproben bestimmter Ammoniumgehalt des Bodens im Vergleich zu dem mittels einer Modellierungssoftware (AgroC) errechneten Ammoniumgehalt des Bodens

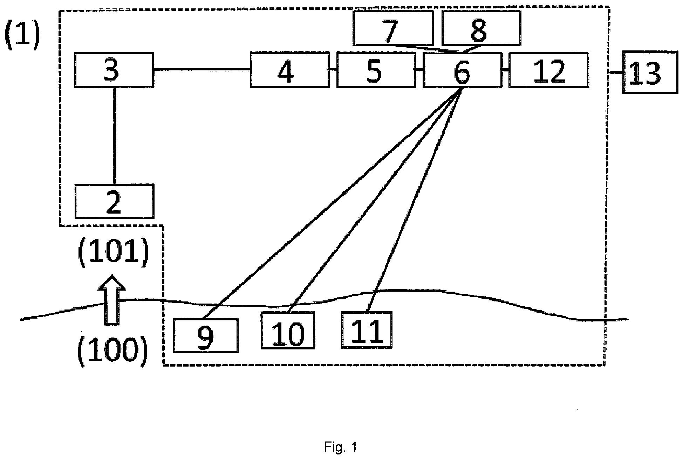

Figur 1 zeigt die offenbarte Messanordnung (1) zur Bestimmung und Überwachung des Ammonium- und Nitrit-Gehalts im Boden (100) anhand von Bodenausgasungen (101). Diese Bodenausgasungen (101) werden mittels einer Gasentnahmevorrichtung (2) und einer Pumpe für Gase (3) zum Multigasmessgerät (5) befördert. Die Förderleistung der Pumpe (3) wird anhand eines Massenflussmessgeräts (4) gemessen und überwacht.

Das Multigasmessgerät (5) misst die Ammoniak ($NH_3$)- und Stickstoffmonoxid (NO)-Konzentration der Bodenausgasungen (101). Anhand einer Auslesevorrichtung (6), die sowohl die Daten des Multigasmessgeräts (5), des Massenflussmessgeräts (4) und die Angabe über die von der Gasentnahmevorrichtung (2) abgedeckte Fläche des Bodens (100) berücksichtigt, werden die Ausgasungsraten von $NH_3$ und NO bestimmt. Anhand einer Datenbank (7), welche zuvor für den jeweiligen Boden ermittelte Referenzdaten beinhaltet, kann die Auswertevorrichtung (8) anhand der ermittelten Ausgasungsraten f sowie optional mit Hilfe von Messdaten weiterer Sensoren (pH-Sensor (9), Temperatursensor (10), Bodenwassergehaltsensor (11), die in den Boden während eines Messvorgangs temporär eingeführt werden, den Ammonium- und den Nitrit-Gehalt des Bodens (100) berechnen. Zur Navigation der Messanordnung und zur räumlichen Zuordnung der Ergebnisse der Bodenausgasungen kann beispielsweise ein GPS-basiertes Positionsbestimmungs- und Navigationssystem verwendet (12) werden. Zum Betrieb der Messanordnung (1) wird die Stromversorgung (13) mittels mobiler Batterie-Einheiten und/oder durch Anschluss an die Batterie eines Fahrzeugs, auf dem die Messanordnung (1) angeordnet ist, und Zwischenschaltung eines DC/AC-Wandlers gewährleistet.

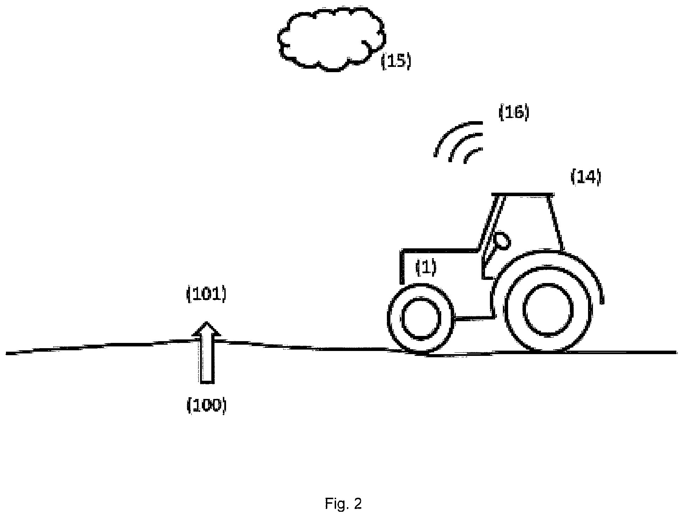

Wie Figur 2 zeigt, kann die Messanordnung (1) alternativ auch an ein Fahrzeug (14), wie beispielsweise einen Traktor, angeordnet und angeschlossen werden, welches über den Boden (100) fährt und mit Hilfe der angeschlossenen Messanordnung (1) die Bodenausgasungen (101) aufnimmt und die qualitative und quantitative Ermittlung des Ammonium-Gehalts und des Ammonium-Umsatzes zu Nitrit im Boden In-situ und in Echtzeit durchführt. Die ermittelten Ergebnisse können dabei auch über eine drahtlose Datenübertragung (16), beispielsweise über Internetverbindung, an einen externen Datenspeicher (15), zum Beispiel eine Daten-Cloud übermittelt werden. Von diesen Ergebnissen kann dann in Echtzeit Gebrauch gemacht werden und damit beispielsweise andere Systeme gesteuert werden (z.B. Düngerstreuer).

Im Folgenden wird beispielhaft, jedoch nicht darauf beschränkt, eine rechnerische Emittlung des Ammonium-Gehalts des Bodens aus den Daten der $NH_3$-Bodenausgasungen, die mit Hilfe der Messanordnung und des erfindungsgemäßen Verfahrens ermittelt wurden, beschrieben.

Zur rechnerischen Ermittlung des Ammonium- und des Nitrit-Gehalts des Bodens mittels der Auswertung der Daten aus den Bodenausgasungen können Modellierungsprogramme (= Modellierungssoftware) eingesetzt werden, die zur Bestimmung von Veränderungen der Konzentrationen relevanter Stoffe im Boden bekannt sind und/oder weiter an die Parameter des Bodens, für den die Veränderungen der Konzentrationen relevanter Stoffe bestimmt werden sollen, angepasst werden.

Die bereits für die Untersuchung des Bodens in der Fachwelt bekannte Modellierungssoftware ermöglicht es, Aussagen über den Umsatz und den Abbau von gelösten Stoffen, insbesondere Stickstoff, Kohlenstoff und Pestiziden in der bioaktiven Zone nahe der Bodenoberfläche (ungefähr vom oberen Ende des Kronendachs bis zum unteren Ende der (historischen) Wurzelzone) zu treffen. Der Umsatz organischer Stoffe im Boden basiert dabei auf mehreren Pools mit Umsatzraten, die von Temperatur, Feuchtigkeit und Tongehalt des Bodens beeinflusst werden.

Die meisten Umsatz- und Abbauprozesse im Boden können mit verschiedenen Teilmodellen der Modellierungssoftware unterschiedlicher Komplexität beschrieben werden, je nach den verfügbaren Daten und den Anforderungen der Nutzer.

Mit Hilfe dieser Modellierungssoftware kann aus den erfindungsgemäß gemessenen Daten der Bodenausgasungen qualitativ und quantitativ In-situ und in Echtzeitermittlung der Ammonium-Gehalt und der Ammonium-Umsatz zu Nitrit im Boden errechnet werden. Als geeignete Modellierungssoftware können beispielsweise, die in der Fachwelt bekannten Modelle, AgroC [1] oder DNDC [2] genannt werden.

Mit der Modellierungssoftware können für den nulldimensionalen Fall alle organischen und anorganischen Nähstoff-Pools des Bodens, wie beispielsweise auch der Ammoniumpool $C_{NH4,t}$, über eine Abbaufunktion 1. Ordnung beschrieben werden. Unter der Bezeichnung "nulldimensionaler Fall" wird das Nicht-Vorhandensein einer räumlichen Auflösung der Bodenparameter über die Tiefe des Bodens verstanden.

Für den Ammoniumpool $C_{NH4,t}$ kann diese Abbaufunktion 1. Ordnung wie folgt beschrieben werden:

$$C_{NH4,t} = C_{NH4,t-1} \cdot e^{\left(-k\Delta_t\varphi_{mf}\right)} \text{ (I)}$$

mit

| | |
|---|---|
| $C_{NH4,i}$: | NH$_4$ Ausgangspoolkonzentration [kg/cm$^2$] |
| $C_{NH4,t-1}$: | NH$_4$ Poolkonzentration [kg/cm$^2$], die zum Zeitpunkt *t-1* nach der Ausgangspoolkonzentration $C_{NH4,t}$ durch Abbau des Ammonium entsteht |
| $k$: | Ausgasungsratenkonstante des Ammonium-Abbaus bei optimalen Bedingungen [1/Tag] |
| $\Delta t$: | zeitlicher Messabstand, zwischen den Messungen t und t-1, der individuell gewählt werden kann und dem Modell je nach gewünschter Einstellung vorgegeben wird |
| $\varphi_{mf}$: | dimensionsloser Faktor, mit dem Abweichungen der Bodenparameter von den optimalen Abbau-Bedingungen für Ammonium im Boden berücksichtigt werden, z.B. niedrigere Bodentemperaturen oder Bodenfeuchte. *m* und *f* kommen aus dem Englischen (rate modifying factors). Sowohl die Bodenfeuchte, als auch die Bodentemperatur werden im Modell berechnet, und aus diesen Daten resultiert dann jeweils ein $\varphi_{mf}$ |

Ammonium wird im Boden abgebaut und wandelt sich zu einem Teil in Ammoniak (NH$_3$) um, welches als Gas aus dem Boden ausgast. Dabei kann die Ausgasungsratenkonstante k beispielsweise aus Literaturwerten bestimmt werden oder vorzugsweise, da genauer als ein Standardwert aus der Literatur, aus bodenspezifischen Daten aus eigenen Abbauversuchen im jeweils untersuchten Boden. Dieser Wert für k wird dann in die obige Formel (I) eingesetzt.

Die Ausgasungsrate $f_{NH3,t}$ [kg/cm$^2$/Tag] gibt den Abbau von Ammonium im Boden zu Ammoniak bezogen auf die Zeit und gemessen als Bodenausgasung wieder.

Dieser Abbau kann durch folgende Gleichung beschrieben werden:

$$f_{NH3,t} = \frac{C_{NH4,t-1} - C_{NH4,t}}{\Delta_t} \qquad \text{(II)}$$

Eingesetzt in die erste Gleichung (I) ergibt sich somit:

$$f_{NH3,t} = \frac{C_{NH4,t-1} - C_{NH4,t-1} * e^{\left(-k\Delta_t\varphi_{mf}\right)}}{\Delta_t} \qquad \text{(III)}$$

$\Delta t$ ist im Modell vorgegeben und $\varphi_{mf}$ wird aus der aktuellen Bodenfeuchte und Bodentemperatur errechnet.

Wenn der Abbau von Ammonium im Boden zu Ammoniak bezogen auf die Zeit und gemessen als Bodenausgasung über die Ausgasungsrate $f_{NH3,t}$ bekannt ist, sowie die Ammoniumpoolgrösse im jeweiligen Boden, kann die Ausgasungsratenkonstante k errechnet werden, wie in dem nachfolgend aufgeführten Beispiel A. Sind aber, wie beim erfindungsgemäßen Verfahren, der Ammoniakfluss durch Bestimmung der Bodenausgasungen und die Ausgasungsratenkonstante k bekannt, kann der Ammoniumpool invers errechnet werden.

Da es unter Feldbedingungen allerdings ein Tiefenprofil der Variablen, wie zum Beispiel der Bodenfeuchte, der Bodentemperatur oder auch des Ammoniumpools, im Boden gibt, wird ein 1-dimensionaler Fall angenommen, d.h. unterschiedliche Werte in unterschiedlichen Bodentiefen, und eine analytische Lösung ist nicht mehr möglich, weil man nur einen Zahlenwert für die Ausgasungsratenkonstante k für das gesamte Profil hat, aber mehrere Unbekannte (>2, z. B. > 2 Ammoniumpoolgrößen) über die Profiltiefe hat. In diesem Fall wird stattdessen ein iteratives Verfahren, eine Modellinversion, angewendet, um die Ammoniumpoolgröße zu bestimmen. Die Differenz in der Ammoniumpoolgröße zwischen $t$ und $t$-$1$ ist dabei in der Praxis irrelevant, da die Ausgasung (oder der Abbau) innerhalb eines Tages ($\Delta t$) in Relation zur Poolgröße so gering ist, dass diese Differenz vernachlässigt werden kann.

Beispiel A: Inverse Bestimmung der Ausgasungsratenkonstante $k$ bei bekannter Ammoniumpoolgröße durch Düngung des Bodens mit 90 kg/ha Ammonium (initiale Ammoniumpoolgröße) und analytisch gemessenen Ausgasungsraten $f_{NH3,t}$ , zeitlicher Messabstand $\Delta t$: 1 Stunde.

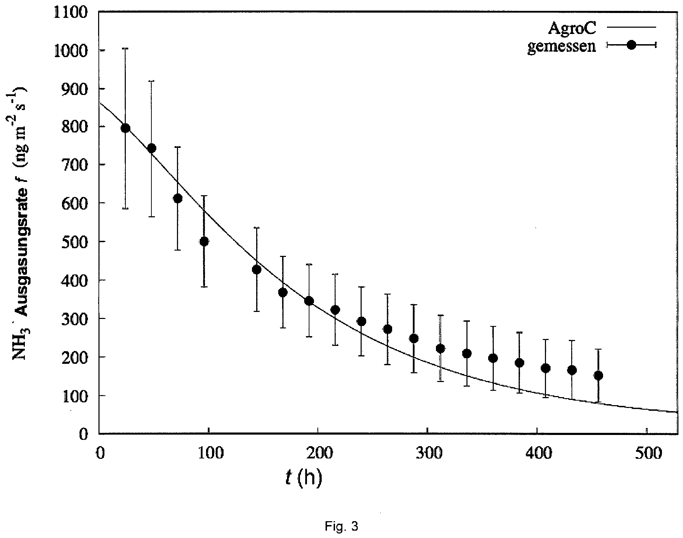

In diesem Beispiel wurde ein Boden mit einer Menge von 90 kg/ha Ammonium gedüngt, so dass hier die Ammoniumpoolgröße bekannt ist. Anschließend wurde in zeitlichen Messabständen $\Delta t$ von 1 Stunde der Ammoniakgehalt der Bodenausgasungen bestimmt und so die Ausgasungsraten $f_{NH3,t}$ bestimmt. Aus den gemessenen Ausgasungsraten zusammen mit der bekannten Ammoniumpoolgröße konnte die Ausgasungsratenkonstante k für dieses Bodenmodell invers bestimmt werden. Mit Hilfe der so ermittelten Ausgasungsratenkonstante $k$ wurde über die Modellierungssoftware AgroC der Abbau des Ammoniums im Boden model-liert. In Figur 3 sind die gemessenen Ausgasungsraten $f_{NH3,t}$ (Y-Achse: $NH_3$ Ausgasungsrate f [ng m$^{-2}$ s$^{-1}$]) und der über AgroC modellierte Ammoniumgehalt des Bodens in Abhängigkeit von den zeitlichen Messabständen (X-Achse: Zeit t [h]) gegeneinander aufgetragen.

Beispiel B: Vergleich von analytisch bestimmtem Ammoniumgehalt des Bodens mit modelliert bestimmtem Ammoniumgehalt des Bodens.

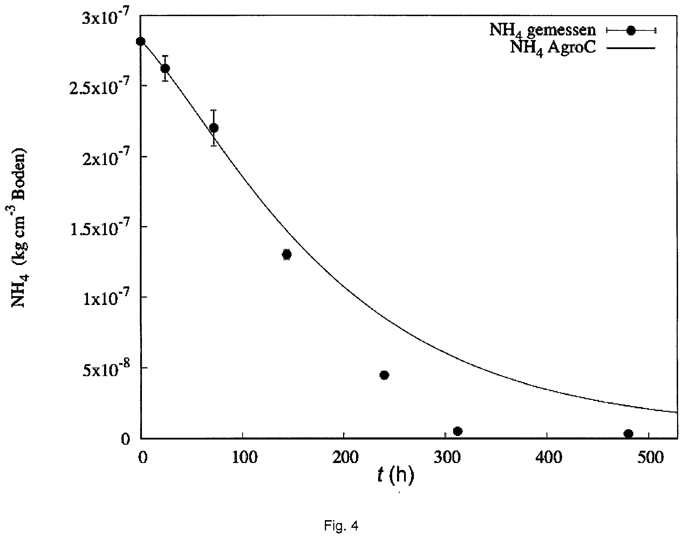

In diesem Beispiel wurden aus dem Boden aus Beispiel A in Abständen von 24 Stunden Bodenproben entnommen und der Ammoniumgehalt dieser Bodenproben jeweils analytisch bestimmt. Die Ergebnisse des analytisch bestimmten Ammoniumgehalts des Bodens wurden mit den Ergebnissen des modellierten Ammoniumgehalts aus den Beispiel A verglichen. In Figur 4 sind die analytisch bestimmten Werte des Ammoniumgehalts (Y-Achse: NH4 [kg cm-3 Boden]) in Abhängigkeit von den zeitlichen Abständen der Probenahme (X-Achse: Zeit t [h]) zusammen mit den über die Modellierung bestimmten Werten des Ammoniumgehalts des Bodens aufgetragen. Wie aus Figur 4 erkennbar ist, stimmen im Wesentlichen die aus den Bodenproben analytisch bestimmten Werte des Ammoniumgehalts mit den über die Modellierung bestimmten Werten überein.

Die Ergebnisse dieser Beispiele zeigen, dass über die Bestimmung des Ammoniakgehalts aus den Bodenausgasungen eine Aussage über den Ammoniumgehalt und den erfolgten Ammoniumabbau im Boden getroffen werden kann. Für die Bestimmung des NO-Gehalts aus den Bodenausgasungen kann dem entsprechend auch eine Aussage über den Nitritgehalt im Boden getroffen werden.

Bezugszeichenliste:

1:   Messanordnung
2:   Gasentnahmevorrichtung
3:   Pumpe
4:   Massenflussgerät
5:   Multigasmessgerät
6:   Auslesevorrichtung
7:   elektronische Datenbank
8:   Auswertevorrichtung
9:   pH-Sensor
10:   Temperatursensor
11:   Bodenwassergehaltssensor
12:   GPS basiertes Positionsbestimmungs- und Navigationssystem
13:   Stromversorgung
14:   Fahrzeug
15:   externer Datenspeicher
16:   drahtlose Datenübertragung

Literaturquellen:

[1] Klosterhalfen, A., Herbst, M., Weihermüller, L., Graf, A., Schmidt, M., Stadler, A., Schneider, K., Subke, J.-A., Huisman, J.A., Vereecken, H., 2017. Multi-site calibration and validation of a net ecosystem carbon exchange model for croplands. Ecological Modelling 363:137-156. http://dx.doi.org/10.1016/j.ecolmodel.2017.07.028

[2] Donna L. Giltrap, Changsheng Li, Surinder Saggar, 2010. DNDC: A process-based model of greenhouse gas fluxes from agricultural soils. Agriculture Ecosystems & Environment 136(3-4): 292-300. DOI: 10.1016/j.agee.2009.06.014

US 16/466,128

**Patentansprüche**

1. Verfahren zur quantitativen In-situ- und Echtzeitermittlung des Ammonium- und Nitrit-Gehalts des Bodens und zur qualitativen Echtzeitermittlung des Ammonium-Umsatzes zu Nitrit im Boden, bei der eine Messanordnung (1) eingesetzt wird, umfassend wenigstens folgende Komponenten:

   eine oder mehrere Gasentnahmevorrichtung/en (2),
   wenigstens ein Multigasmessgerät (5),
   wenigstens ein Massenflussgerät (4),
   wenigstens eine Auslesevorrichtung (6) sowie
   wenigstens eine Auswertevorrichtung (8) für das Multigasmessgerät (5), umfassend folgende Verfahrensschritte:

   a) Entnahme von Bodenausgasungen (101) eines Bodens (100) mittels einer oder mehrerer Gasentnahmevorrichtung/en (2) und Beförderung dieser Bodenausgasungen (101) zu wenigstens einem Multigasmessgerät (5),
   b) Bestimmung des Massenflusses der Bodenausgasungen (101) zum Multigasmessgerät (5) anhand eines Massenflussmessgeräts (4),
   c) Bestimmung der Ausgasungsraten $f$ von $NH_3$ und NO aus dem Boden (100) mit einer Auslesevorrichtung (6) aus den Daten des Multigasmessgeräts (5) und des Massenflussmessgeräts (4), bezogen auf die von der Gasentnahmevorrichtung (2) abgedeckte Fläche des Bodens (100),
   d) Rechnerische Bestimmung des Ammonium- und des Nitrit-Gehalts des Bodens (100) durch eine Auswertevorrichtung (8), wobei für die Bestimmung des Ammonium-Gehalts die ermittelte Ausgasungsrate $f$ von $NH_3$ und für die Bestimmung des Nitrit-Gehalts die ermittelte Ausgasungsrate $f$ von NO aus Schritt c) mit zuvor für den Boden (100) jeweils ermittelten Kalibrationsdaten verglichen wird und so In-situ die lokale Konzentration des Ammonium- und des Nitrit-Gehalts des Bodens (100) quantitativ sowie der Umsatz von Ammonium zu Nitrit qualitativ in Echtzeit bestimmt werden.

2. Verfahren nach vorhergehendem Anspruch,
   **dadurch gekennzeichnet, dass** die Entnahme von Bodenausgasungen (101) an wenigstens zwei unterschiedlichen Positionen des Bodens (100) mit Hilfe von wenigstens zwei Gasentnahmevorrichtungen (2) erfolgt und die Bestimmung der räumlichen Zuordnung der Ergebnisse dieser Bodenausgasungen (101) über ein GPS-basiertes Positionsbestimmungs- und Navigationssystem (12) erfolgt.

3. Verfahren nach einem der vorhergehenden Ansprüche, **dadurch gekennzeichnet, dass** die Messanordnung (1) zu unterschiedlichen Positionen des Bodens (100) bewegt wird.

4. Verfahren nach einem der vorhergehenden Ansprüche, **dadurch gekennzeichnet, dass** die Entnahme von Bodenausgasungen (101) an einer Vielzahl von unterschiedlichen Positionen im Boden (100) erfolgt und die Bestimmung der räumlichen Zuordnung der Messwerte und Ergebnisse dieser Bodenausgasungen (101) über ein GPS-basiertes Positionsbestimmungs- und Navigationssystem (12) erfolgt.

5. Verfahren nach einem der vorhergehenden Ansprüche, **dadurch gekennzeichnet, dass** Messdaten weiterer Sensoren, insbesondere pH-Sensoren (9), Temperatursensoren (10), Bodenwassergehaltsensoren (11), die in den Boden (100) während eines Messvorgangs temporär eingeführt werden, mit zuvor für den Boden ermittelten Kalibrationsdaten verglichen werden und als Parameter bei der rechnerischen Bestimmung des Ammonium- und des Nitrit-Gehalts gemäß Schritt d) berücksichtigt werden.

6. Verfahren nach einem der vorhergehenden Ansprüche, **dadurch gekennzeichnet, dass** die Ergebnisse aus Verfahrensschritt d) extern gespeichert und abgerufen werden.

7. Verfahren nach einem der vorhergehenden Ansprüche, **dadurch gekennzeichnet, dass** die gesamten Komponenten der Messanordnung (1) mit Hilfe eines Fahrzeugs an die unterschiedlichen Positionen des Bodens bewegt werden.

**Claims**

1. A method for the qualitative and quantitative in-situ, real-time determination of the ammonium and nitrite content and the ammonium conversion to nitrite in soil, wherein a measuring arrangement (1) is used which comprises at least the following components:

    one or more gas sampling devices (2),
    at least one multi-gas measuring device (5),
    at least one mass flow device (4),
    at least one readout device (6), and
    at least one evaluation device (8) for the multi-gas measuring device (5), comprising the following method steps:

        a) sampling soil emissions (101) from a soil (100) by means of one or more gas sampling devices (2) and transporting these soil emissions (101) to at least one multi-gas measuring device (5),
        b) determining the mass flow of the soil emissions (101) to the multi-gas measuring device (5) using a mass flow measuring device (4),
        c) determining the gas emission rates f of $NH_3$ and NO from the soil (100) using a readout device (6) from the data of the multi-gas measuring device (5) and the mass flow measuring device (4) relative to the surface area of the soil (100) covered by the gas sampling device (2),
        d) calculation of the ammonium and nitrite content of the soil (100) by means of an evaluation device (8), wherein, in order to determine the ammonium content, the determined gas emission rate f of $NH_3$ is compared with calibration data previously determined for the soil (100) and, in order to determine the nitrite content, the determined gas emission rate f of NO from step c) is compared with calibration data previously determined for the soil (100), whereby the local concentration of the ammonium and nitrite content of the soil (100) is determined quantitatively in situ and the conversion of ammonium to nitrite is determined qualitatively in real time.

2. The method according to the preceding claim, **characterized in that** the sampling of soil emissions (101) is performed at at least two different positions on the soil (100) with the aid of at least two gas sampling devices (2), and the determination of the spatial allocation of the results for these soil emissions (101) is performed using a GPS-based positioning and navigation system (12).

3. The method according to any one of the preceding claims, **characterized in that** the measuring arrangement (1) is moved to different positions on the soil (100).

4. The method according to any one of the preceding claims, **characterized in that** the sampling of soil emissions (101) is performed at a plurality of different positions in the soil (100) and the determination of the spatial allocation of the measured values and results for these soil emissions (101) is performed using a GPS-based positioning and navigation system (12).

5. The method according to any one of the preceding claims, **characterized in that** measurement data from other sensors, in particular pH sensors (9), temperature sensors (10), soil water content sensors (11), which are temporarily introduced into the soil (100) during a measurement process, are compared with calibration data previously determined for the soil and are taken into account as parameters in the computation of the ammonium and nitrite content according to step d).

6. The method according to any one of the preceding claims, **characterized in that** the results from method step d) are stored and retrieved externally.

7. The method according to any one of the preceding claims, **characterized in that** all of the components of the measuring arrangement (1) are moved to different positions on the soil with the aid of a vehicle.

**Revendications**

1. Procédé de détermination quantitative in situ et en temps réel de la teneur du sol en ammonium et en nitrite et de détermination qualitative en temps réel de la transformation de l'ammonium en nitrite dans le sol, dans lequel on utilise un agencement (1) de mesure comprenant au moins les composants suivants :

   un ou plusieurs dispositif/s (2) de prélèvement de gaz,
   au moins un appareil (5) de mesure de plusieurs gaz,
   au moins un appareil (4) d'écoulement massique,
   au moins un dispositif (6) de lecture ainsi qu' au moins un dispositif (8) d'évaluation pour l'appareil (5) de mesure à plusieurs gaz, comprenant les stades de procédé suivants :

   a) prélèvement de dégagements (101) de gaz du sol (100) au moyen d'un ou de plusieurs dispositif/s (2) de prélèvement de gaz et transport de ces dégagements (101) du sol au moins un appareil (5) de mesure de plusieurs gaz,
   b) détermination de l'écoulement massique des dégagements (101) du sol à l'appareil (5) de mesure à plusieurs gaz, à l'aide d'un appareil (4) de mesure de l'écoulement massique,
   c) détermination des taux f de dégagements de $NH_3$ et NO du sol (100) par un dispositif (6) de lecture, à partir des données de l'appareil (5) de mesure de plusieurs gaz et de l'appareil (4) de mesure de l'écoulement massique, rapporté à la surface du sol (100) recouverte par le dispositif (2) de prélèvement de gaz,
   d) détermination informatique de la teneur du sol (100) en ammonium et en nitrite par un dispositif (8) d'évaluation, dans lequel, pour la détermination de la teneur en ammonium, on compare, à des données d'étalonnage, déterminées respectivement auparavant pour le sol (100), pour la détermination de la teneur en ammonium, le taux f de dégagement déterminé d'$NH_3$ et, pour la détermination de la teneur en nitrite, le taux f de dégagement déterminé de NO, dans le stade c) et on détermine ainsi en temps réel in situ quantitativement la concentration de la teneur du sol (100) en ammonium et en nitrite, ainsi que qualitativement la transformation d'ammonium en nitrite.

2. Procédé suivant la revendication précédente, **caractérisé en ce que** le prélèvement de dégagements (101) de gaz du sol s'effectue en au moins deux positions différentes du sol (100) à l'aide de deux dispositifs (2) de prélèvement de gaz et la détermination de l'affectation dans l'espace des résultats de ces prélèvements (101) de gaz du sol s'effectue par un système (12) de détermination de position ou de navigation reposant le GPS.

3. Procédé suivant l'une des revendications précédentes, **caractérisé en ce que** l'on déplace l'agencement (1) de mesure en des positions différentes du sol (100).

4. Procédé suivant l'une des revendications précédentes, **caractérisé en ce que** le prélèvement de dégagements (101) de gaz du sol s'effectue en une pluralité de positions différentes du sol (100) et la détermination de l'affectation dans l'espace des valeurs de mesure et des résultats de ces dégagements (101) de gaz du sol s'effectue par un système (12) de détermination de position et de navigation reposant sur le GPS.

5. Procédé suivant l'une des revendications précédentes, **caractérisé en ce que** l'on compare, à des données d'étalonnage déterminées auparavant pour le sol, des données de mesure d'autres capteurs, en particulier de capteurs (9) de pH, de capteurs (10) de température, de capteurs (11) de teneur en eau du sol, que l'on a insérés temporairement dans le sol (100) pendant une opération de mesure, et on les prend en compte comme paramètres dans la détermination informatique de la teneur en ammonium et en nitrite suivant le stade d).

6. Procédé suivant l'une des revendications précédentes, **caractérisé en ce que** l'on met en mémoire à l'extérieur et on appelle les résultats du stade d) du procédé.

7. Procédé suivant l'une des revendications précédentes, **caractérisé en ce que** l'on déplace, à l'aide d'un véhicule sur les positions différentes du sol, l'ensemble des composants de l'agencement (1) de mesure.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 16466128 B

**In der Beschreibung aufgeführte Nicht-Patentliteratur**

- **WALKER JOHN T et al.** Nitrogen trace gas emissions from a riparian ecosystem in southern Appalachia. *Chemosphere*, 02 November 2002, vol. 49 (10), ISSN 0045-6535, 1389-1398
- **KLOSTERHALFEN, A.** ; **HERBST, M.** ; **WEIHERMÜLLER, L.** ; **GRAF, A.** ; **SCHMIDT, M.** ; **STADLER, A.** ; **SCHNEIDER, K.** ; **SUBKE, J.-A.** ; **HUISMAN, J.A.** ; **VEREECKEN, H.** Multi-site calibration and validation of a net ecosystem carbon exchange model for croplands. *Ecological Modelling*, 2017, vol. 363, 137-156, http://dx.doi.org/10.1016/j.ecolmodel.2017.07.028
- **DONNA L. GILTRAP** ; **CHANGSHENG LI** ; **SURINDER SAGGAR**. DNDC: A process-based model of greenhouse gas fluxes from agricultural soils. *Agriculture Ecosystems & Environment*, 2010, vol. 136 (3-4), 292-300